## Europäisches Patentamt

## European Patent Office

## Office européen des brevets

(11) Veröffentlichungsnummer: **0 023 994**
**B1**

(12) # EUROPÄISCHE PATENTSCHRIFT

(45) Veröffentlichungstag der Patentschrift:
18.04.84

(51) Int. Cl.³: **A 61 K 6/00**

(21) Anmeldenummer: 80104339.9

(22) Anmeldetag: 23.07.80

(54) Zahnmedizinische Wurzelfüllpaste.

(30) Priorität: 13.08.79 DE 2932738

(43) Veröffentlichungstag der Anmeldung:
18.02.81 Patentblatt 81/7

(45) Bekanntmachung des Hinweises auf die Patenterteilung:
18.04.84 Patentblatt 84/16

(84) Benannte Vertragsstaaten:
AT BE CH FR GB IT LI LU NL SE

(56) Entgegenhaltungen:
DE - A - 2 629 694
US - A - 2 516 438
US - A - 3 205 132
US - A - 3 266 147

CHEMICAL ABSTRACTS, Band 92, Nr. 22, 2. Juni 1980,
Seite 296, Nr. 185845v, Columbus, Ohio, U.S.A., U.
DELBENE et al.: "Pharmaceutical and cosmetic
applications of neats-foot oil and its fractions"
CHEMICAL ABSTRACTS, Band 51, Nr. 22, 25. November
1957, Spalte 18477b, Columbus, Ohio, U.S.A., U. BOGS
et al.: "Knowledge of Oleum pedum tauri (neat's-foot
oil)"
I. GRUHN et al. Stomatol. DDR 28 (1978) S. 569-575

(73) Patentinhaber: **Dietz, Georg, Dr., Mauerkircher
Strasse 120, D-8000 München 81 (DE)**
Patentinhaber: **Barth, Hans-Helmut, Dr. Dr.,
Jäcklinstrasse 21, D-8000 München 83 (DE)**

(72) Erfinder: **Dietz, Georg, Dr., Mauerkircher Strasse 120,
D-8000 München 81 (DE)**
Erfinder: **Barth, Hans-Helmut, Dr. Dr., Jäcklinstrasse 21,
D-8000 München 83 (DE)**

(74) Vertreter: **Patentanwälte Henkel, Pfenning, Feiler,
Hänzel & Meinig, Möhlstrasse 37,
D-8000 München 80 (DE)**

Zahnmedizinische Wurzelfüllpaste

Die Erfindung betrifft eine zahnmedizinische Wurzelfüllpaste mit Calciumhydroxid als wirksamem Bestandteil, einem Röntgenkontrastmittel und einer Pastengrundlage.

Die Behandlung der chronisch-periapikalen Parodontitis und der Gangrän gilt auch heute noch als besonders kompliziert, wobei zudem die Erfolgsquote der Behandlung relativ gering ist. Die niedrige Erfolgsquote bei der Behandlung der Gangrän und chronisch-periapikalen Parodontitis ist vornehmlich darauf zurückzuführen, dass keine vollständige Beseitigung der massiven bakteriellen Infektion erreicht wird und eine Umstimmung und Heilung des akut oder chronisch entzündeten periapikalen Gewebes ausbleibt. Für eine erfolgreiche Behandlung der Gangrän und chronisch-periapikalen Parodontitis ist es unabdingbar, neben einer vollständigen Wurzelfüllung eine möglichst gute medikamentöse Versorgung des Apex und des periapikalen Gewebes sicherzustellen, um das saure Milieu im Bereich der chronisch entzündeten periapikalen Gewebes zur Induktion der osteoregenerierenden Phase umzustimmen.

Zur Füllung gangränöser Zähne mit chronisch-periapikaler Parodontitis stehen eine grosse Zahl verschiedener Medikamente zur Verfügung. Die wichtigsten von ihnen und in der westlichen Welt am meisten verwendeten sind die aus einer Chlorphenol/Kampfer/Menthol/Jodoform-Paste bestehende Walkhoff'sche Paste (vgl. O. Walkhoff «Mein System der medikamentösen Behandlung schwerer Erkrankungen der Zahnpulpa und der Periodontiums», Verlag Meuser, Berlin 1928), die polyantibiotische Paste auf der Basis verschiedener Antibiotika und eines flüssigen Silikons als Pastengrundlage (vgl. L.I. Grossmann «Lehrbuch der modernen Wurzelbehandlung» Medica Verlag Stuttgart 1968), die Wurzelfüllpaste N2 nach A. Sargenti (vgl. A. Sargenti «Rationelle Wurzelbehandlung» Quintessenz-Verlag 1968) und die von B. W. Hermann entwickelte Wurzelfüllpaste auf Calciumhydroxidbasis (vgl. B. W. Hermann in «Zahnärztl. Rdsch.», Band 39, Seite 888 [1930] und Medizinische Dissertation Würzburg 1920 «Calciumhydroxid als Mittel zum Behandeln und Füllen von Zahnwurzelkanälen»).

Nachteilig an der Walkhoff'schen Paste ist, dass sie nach ihrer Applikation im Laufe der Zeit resorbiert wird und somit tote Räume entstehen. Die Walkhoff'sche Paste ist somit lediglich als temporäres resorbierbares Füllmaterial anzusehen. Offensichtliche Probleme bei der Anwendung der polyantibiotischen Paste bestehen in allergischen Reaktionen und Resistenzbildungen bei chronisch persistierenden Erregern. Problematisch an der Wurzelfüllpaste N2 ist deren nahe an der Toxizitätsgrenze von 5% (vgl. E. Sauerwein «Zahnerhaltungskunde» Thieme-Verlag Stuttgart 1972) liegender Gehalt an Paraformaldehyd von 4,7%. Grundsätzlich lässt sich Calciumhydroxid zur Füllung gangränöser Zähne mit chronisch-periapikaler Parodontitis mit gutem Erfolg verwenden, da Calciumhydroxid einerseits die sauren Entzündungsbezirke alkalisiert und das saure Milieu in den Wurzelkanälen ins Alkalische umstimmt, was zur Folge hat, das Entzündungen beseitigt und allmählich eine Hartgewebsbarriere gebildet werden. Ein grosser Nachteil der bisher bekannten Wurzelfüllpasten auf Calciumhydroxidbasis, die beispielsweise Ringerlösung als Pastengrundlage enthalten, ist deren sehr schlechte Einbringbarkeit in die Wurzelkanäle. Dies ist darauf zurückzuführen, dass solche Wurzelfüllpasten offensichtlich infolge des erforderlichen hohen Gehalts an Calciumhydroxid sehr bröselig sind. Eine, wie bereits angedeutet, vollständige Füllung der Wurzelkanäle ist in der Praxis somit praktisch nicht möglich.

Weiterhin bereitet die Feststellung des Füllgrades der Wurzelkanäle bei Anwendung von auf Calciumhydroxid basierenden Wurzelfüllpasten wegen des fehlenden Röntgenkontrasts von Calciumhydroxid Schwierigkeiten. Es gibt zwar im Handel bereits eine zahnmedizinische Wurzelfüllpaste auf Calciumhydroxidbasis mit einem Röntgenkontrastmittel, der Füllgrad der Wurzelkanäle lässt sich aber auch bei Applikation dieser Wurzelfüllpaste offensichtlich wegen des grossen Verhältnisses Calciumhydroxid zu Röntgenkontrastmittel nur schwierig feststellen. Abgesehen davon wird durch den Zusatz des Röntgenkontrastmittels zu der Wurzelfüllpaste aus Calciumhydroxidbasis deren schlechte Einbringbarkeit in die Wurzelkanäle nicht im geringsten verbessert.

Der geringe Röntgenkontrast und die Schwierigkeiten bei der Einbringung von Wurzelfüllpasten auf Calciumhydroxidbasis in die Wurzelkanäle dürften die Hauptgründe dafür sein, dass der Walkhoff'schen Paste vor einer Wurzelfüllpaste auf Calciumhydroxidbasis immer noch der Vorzug gegeben wird (vgl. A. Herforth und M. Strassburg in «Dtsch. zahnärztl. Z.», Band 32, Seiten 453 bis 459 [1977]).

Die US-A 2 516 438 beschreibt ein Abdeckmaterial für Zahnpulpe mit einem Gehalt an Calciumhydroxid gegebenenfalls einem Röntgenkontrastmittel und Eugenol (als Anteigmittel) sowie gegebenenfalls Guajakol und Leinsaatöl. Die US-A 3 266 147 beschreibt ein Zahnfüllmittel zum Auskleiden von Bohrlöchern in Zähnen vor der endgültigen Füllung. Das Mittel besteht aus einer dünnen flexiblem Folie, die möglichst homogen mit Calciumhydroxid und einem Röntgenkontrastmittel imprägniert ist. Die US-A 3 205 132 offenbart eine Wurzelfüllpaste auf wasserfreier Basis mit Calciumhydroxid, einem Röntgenkontrastmittel, fungiziden und bakteriziden Zusätzen und einer hauptsächlich eugenolhaltigen Pastengrundlage (flüssigen Phase).

Aus der DE-OS 2 629 694 ist ein Dentalpräparat, insbesondere auch eine Wurzelfüllpaste bekannt, die neben Calciumhydroxid und einem Röntgenkontrastmittel als Pastengrundlage ein Reaktionsprodukt aus Formaldehyd, einer Phenolverbindung und einem alkohollöslichen pflanzlichen Öl enthält.

Die aus den genannten Literaturstellen bekannten Dentalpräparate sind ebenfalls mit mindestens einem der vorstehend geschilderten Nachteile behaftet und können somit in der Praxis nicht zufriedenstellen.

Der Erfindung lag nun die Aufgabe zugrunde, die bekannten Wurzelfüllpasten auf Calciumhydroxidbasis so weit zu verbessern, dass sie sich ohne Schwierigkeiten in die Wurzelkanäle gangränöser Zähne einbringen lassen, nach ihrer Applikation die Wurzelkanäle vollständig füllen und eine röntgenologische Bestimmung des Füllgrades der Wurzelkanäle ermöglichen.

Gegenstand der Erfindung ist somit eine zahnmedizinische Wurzelfüllpaste mit einem Gehalt an Calciumhydroxid und einem Röntgenkontrastmittel, welche dadurch gekennzeichnet ist, dass sie als Pastengrundlage Oleum pedum tauri enthält. Zweckmässigerweise enthält die Wurzelfüllpaste gemäss der Erfindung als Röntgenkontrastmittel Bariumsulfat.

Eine bevorzugte Wurzelfüllpaste gemäss der Erfindung enthält etwa 55 Gew.-% Calciumhydroxid, etwa 23 Gew.-% Bariumsulfat als Röntgenkontrastmittel und etwa 22 Gew.-% Oleum pedum tauri. Die Mengenanteile der Einzelbestandteile können, je nach den zu füllenden Zähnen, allgemein ± 10, zweckmässigerweise ± 5, vorzugsweise ± 2% schwanken. Hierbei wird in der Regel bei Frontzähnen mit mehr Calciumhydroxid, bei Molaren — um einen ausreichenden Röntgenkontrast auch im Seitenzahnbereich zu erreichen — mit mehr Bariumsulfat auf Kosten von Calciumhydroxid gearbeitet. Insbesondere ist zu beachten, dass einerseits der Calciumhydroxidanteil möglichst hoch sein soll, andererseits aber auch die Untergrenze an Bariumsulfat durch einen noch eindeutig identifizierbaren Röntgenkontrast gegeben ist.

Aus «Chemical Abstracts», Band 92, Nr. 188477b ist die Verwendbarkeit von Oleum pedum tauri zum Härten pharmazeutischer Zubereitungen anstelle von hydriertem Erdnussöl, aus «Chemical Abstracts» Nr. 185845y die Verwendung von Oleum pedum tauri als lipophiles Streckmittel in Arzneimittel- und kosmetischen Zubereitungen bekannt. Die Verwendung von Oleum padum tauri als Pastengrundlage in Wurzelfüllpasten ist dadurch aber nicht nahegelegt. Oleum pedum tauri wurde auch bereits in der Zahnheilkunde eingesetzt (vgl. I. Gruhn, G. Klinger und G. Lange «Prüfung der desinfizierenden Wirkung des Calciumperoxids als Wurzelkanalfüllmaterial» Stomatol DDR 28 [1978]). In der genannten Literaturstelle wird über die desinfizierende Wirkung von Calciumperoxid auf den Wurzelkanal und die periapikale Region berichtet.

Durch die gemeinsame Verwendung von Calciumperoxid/Oleum pedum tauri soll die plötzliche Spaltung des Peroxids und dadurch die Möglichkeit der überdruckbedingten periapikalen Entzündung ausgeschlossen werden. Dies trifft jedoch, wie die später wiedergegebenen einschlägigen Versuchsergebnisse zeigen, nicht zu. Bei der klinischen Prüfung der «medizinischen Wurzelfüllpaste» Calciumperoxid/Oleum pedum tauri ist bei 80% der Patienten eine Woche nach der Wurzelfüllung noch ein auf eine Exazerbation zurückzuführender periapikaler Druckschmerz festzustellen, d.h. die Kombination Calciumperoxid und Oleum pedum tauri eignet sich entgegen der Aussage in der genannten Literaturstelle nicht als zahnmedizinische Wurzelfüllpaste.

Es bestand somit für den Fachmann nicht die geringste Veranlassung, den einen Bestandteil der bekannten Kombination, nämlich das Oleum pedum tauri, dem in der bekannten Kombination die letztlich doch nicht gelöste Aufgabe zukommt, die Sauerstoffabspaltung aus dem Calciumperoxid so weit zu verlangsamen, dass es nicht mehr zu einer überdruckbedingten periapikalen Entzündung kommt, mit zwei anderen — zugegebenermassen bekannten — Bestandteilen einer zahnmedizinischen Wurzelfüllpaste, nämlich Calciumhydroxid und einem Röntgenkontrastmittel, zu kombinieren. Es war für den Fachmann in Kenntnis der genannten Literaturstelle und der beim Nacharbeiten der Lehren dieser Literaturstelle gewonnenen Erkenntnisse nicht vorhersehbar, dass er mit der Kombination Calciumhydroxid, Röntgenkontrastmittel und Oleum pedum tauri überhaupt zu einer in der Praxis einsetzbaren zahnmedizinischen Wurzelfüllpaste gelangen könne, die darüber hinaus auch noch zu einer erheblichen und raschen Schmerzverminderung führt.

Eine Wurzelfüllpaste gemäss der Erfindung lässt sich trotz ihrer hohen Calciumhydroxidkonzentration unmittelbar nach ihrer Zubereitung und während eines gewissen Zeitraums danach ohne Schwierigkeiten in den aufbereiteten Wurzelkanal und Knochendefekt einbringen, da sie während dieses Zeitraums eine glatte Konsistenz aufweist und nicht bröselig ist. Nach der Applikation füllt die Wurzelfüllpaste gemäss der Erfindung die Wurzelkanäle vollständig und lässt ohne Schwierigkeiten wegen ihres guten Röntgenkontrasts eine Feststellung des Füllgrades der Wurzelkanäle und des periapikalen Bereiches zu.

Einer Wurzelfüllpaste gemäss der Erfindung können gegebenenfalls auf dem Gebiet der Zahnheilkunde übliche Zusätze zur Behandlung gangränöser Zähne einverleibt werden. Vielversprechend ist beispielsweise ein Zusatz von Vitamin B bei Neuritiden und Neuralgien.

Das folgende Beispiel soll die Erfindung näher veranschaulichen.

### Beispiel

Unter Verwendung einer Wurzelfüllpaste gemäss der Erfindung wurden bei 33 Patienten im Alter von 18 bis 66 Jahren männlichen bzw. weiblichen Geschlechts 47 Wurzelbehandlungen an Front- und Eckzähnen sowie Prämolaren durchgeführt. Es handelte sich um gangränöse Zähne mit chronisch-apikaler Parodontitis, die sich in einem chronischen oder akut-exazerbativen Stadium befanden. Einn Teil der Zähne war bereits trepaniert, die anderen befanden sich in noch geschlossenem Zustand.

Nach eingehender Anamnese, klinischer Befundaufnahme und röntgenologischer Darstellung mit apikaler Einstellung des jeweils erkrankten Zahnes erfolgte gegebenenfalls nach zahnmedizinisch üblicher Vorbehandlung eine zweiphasige Aufbereitung des Wurzelkanals. Nach erfolgter Reinigung und Desinfektion, Neutralisation und Trocknen der Wurzelkanäle wurde die erfindungsgemässe Wurzelfüllpaste mit Hilfe eines geeigneten zahnärztlichen Instruments in die Wurzelkanäle eingebracht. Hierbei erfolgte auch ein Weitertransport der in die Wurzelkanäle eingebrachten Wurzelfüllpaste über das erweiterte Foramen apicale in das entzündete periapikale Gewebe.

Nach erfolgter Füllung wurde der Füllgrad röntgenologisch kontrolliert. In sämtlichen Fällen zeigte es sich, dass eine korrekte Wurzelfüllung erreicht war.

Letztlich wurde jeweils eine Composit-Füllung gelegt.

Als Wurzelfüllmaterial wurde eine erfindungsgemässe Wurzelfüllpaste der folgenden Zusammensetzung verwendet:

|  |  |  |  | Gew.-% |
|---|---|---|---|---|
| 1. Ca(OH)$_2$ | min. | 96%) | | |
| CaCO$_3$ | | ) | | |
| (Calciumcarbonat) | max. | 3%) | | |
| in Salzsäure unlösliche | | ) | | |
| Anteile | max. | 0,1%) | | |
| | | ) | * | ** |
| Chlorid (Cl) | max. | 0,005%) | 50 - 55 | |
| Sulfat (SO$_4$) | max. | 0,2%) | | |
| Schwermetalle | | ) | | |
| (als Pb) | max. | 0,005%) | | |
| Eisen (Fe) | max. | 0,05%) | | |
| mit Ammoniumoxalat | | ) | | |
| nicht fällbare Anteile | | ) | | |
| als Sulfat | max. | 2,5%) | | |
| | | | * | ** |
| 2. Bariumsulfat (rein) | | | 28 - 23 | |
| 3. Oleum pedum tauri (rein) | | | 22 - 22 | |

\* zur Füllung von Molaren
\*\* zur Füllung von Frontzähnen

Bei fünf behandelten gangränösen Zähnen mit apikaler Parodontitis lag als Komplikation gleichzeitig eine Fistelung ins Vestibulum vor.

Bei diesen Patienten erstreckte sich die Behandlung nicht nur auf die Wurzelfüllung und Füllung des periapikalen Granuloms, sondern gleichzeitig auf die Einbringung des Materials in den Fistelgang. Als sicheres Zeichen für den Applikationserfolg konnte der Austritt des Materials aus dem Fistelgang ins Vestibulum bei transkanalärer Abfüllung angesehen werden. Auch dies zeigt die leichte und sichere Applizierbarkeit der erfindungsgemässen Wurzelfüllmasse.

Aus den Nachuntersuchungen und Kontrollen ergab sich, dass von den 33 behandelten Patienten, von denen 29 zu Behandlungsbeginn über Schmerzen klagten, nach der Behandlung lediglich noch12 während der ersten 24 Stunden meist leichte Schmerzen hatten. Bei nur zwei Patienten hielten die Beschwerden während der ersten Woche nach der Behandlung an, wurden aber zunehmend leichter. Lediglich eine Patientin, die verschiedene schwere Stoffwechselleiden hat, klagte noch nach mehr als einer Woche über gelegentlich auftretende Beschwerden.

Neben der Schmerzanamnese zeigte sich bei Behandlungsbeginn durch Inspektion und Palpation bei 11 Patienten eine mehr oder weniger stark ausgeprägte Schwellung. Diese bildete sich während des ersten Tages nach der Behandlung bei 4 Patienten bereits völlig zurück, bei weiteren 4 innerhalb der ersten Woche. Lediglich 3 Patienten hatten nach Ablauf der ersten Woche noch leichte Schwellungssymptome.

Fünf behandelte Zähne hatten zu Behandlungsbeginn Fistelungen ins Vestibulum. Eine dieser Fisteln verschwand durch die Behandlung bereits nach 4 Wochen, die anderen vier innerhalb von 2 Monaten.

Am Apex liess sich röntgenologisch nach 3, 6, 9 oder 12 Monaten zeigen, dass Aufhellungsrückbildungen stattgefunden hatten.

Bei 9 Zähnen zeigte sich klinisch eine Hartgewebsbarriere und röntgenologisch ein apikaler Verschluss. Diese Zähne wurden endgültig wurzelgefüllt.

Ein Zahn wurde 6 Monate nach der Wurzelbehandlung stiftfixiert. Hier stellte die Wurzelbehandlung lediglich die Vorbereitung auf eine schon zu Behandlungsbeginn geplante Stiftfixation dar.

*Versuchsergebnisse bei einer vergleichenden in-vivo-Behandlung mit Calciumperoxid/Oleum pedum tauri bzw. der Wurzelfüllpaste gemäss der Erfindung*

Jeweils 10 Patienten erhielten eine Wurzelfüllung mit Calciumperoxid/Oleum pedum tauri bzw. der Wurzelfüllpaste gemäss der Erfindung. 80% der mit Calciumperoxid/Oelum pedum tauri behandelten Patienten leiden nach 1 Woche immer noch unter einem auf eine Exazerbation zurückzuführenden periapikalen Druckschmerz. Demgegenüber sind nach 1 Tag bereits 60%, nach 1 Woche mindestens 90% der mit der Wurzelfüllpaste gemäss der Erfindung behandelten Patienten schmerzfrei.

**Patentanspruch**

Zahnmedizinische Wurzelfüllpaste mit einem Gehalt an Calciumhydroxid und einem Röntgenkontrastmittel, dadurch gekennzeichnet, dass sie als Pastengrundlage Oleum pedum tauri enthält.

**Claim**

Root filling paste comprising calcium hydroxide and an X-ray contrast agent, characterized by containing oleum pedum tauri as paste base.

**Revendication**

Pâte dentaire de remplissage des racines contenant de l'hydroxyde de calcium et un agent de contraste aux rayons X, caractérisée en ce qu'elle contient comme base de pâte de l'oleum pedum tauri.